Europäisches Patentamt

European Patent Office

Office européen des brevets

(11) Publication number: **0 522 203 A1**

## EUROPEAN PATENT APPLICATION

(21) Application number: **91201185.5**

(22) Date of filing: **16.05.91**

(51) Int. Cl.5: **C12N 9/52**, C12N 15/57, C12N 1/21, C12P 21/08, A61K 39/395, C12Q 1/37, C12N 15/65, A23C 19/032, A23C 19/06

The applicant has subsequently filed a sequence listing and declared, that it includes no new matter.

(43) Date of publication of application:
**13.01.93 Bulletin 93/02**

(84) Designated Contracting States:
**AT BE CH DE DK ES FR GB GR IT LI LU NL SE**

(71) Applicant: **PROBICOM RESEARCH B.V.
Snekerweg 15
NL-8701 XB Bolsward(NL)**

(72) Inventor: **Tan, Paris Som Tjwan
Vinkenstraat 122a
NL-9713 TM Groningen(NL)**
Inventor: **Konings, Wilhelmus Nicolaas
Rijksstraatweg 236
NL-9752 CJ Haren(NL)**

(74) Representative: **Smulders, Theodorus A.H.J.,
Ir. et al
Vereenigde Octrooibureaux Nieuwe Parklaan 97
NL-2587 BN 's-Gravenhage(NL)**

(54) Protein from lactic acid bacteria.

(57) The present invention relates to an endopeptidase of lactic acid bacteria in isolated or purified form as well as to uses thereof, e.g., in the preparation of foods such as cheese, whippable products and meat products, or for preparing antibodies against the endopeptidase. The invention also relates to recombinant genetic information coding for the endopeptidase and transformed organisms expressing the endopeptidase. The endopeptidase can be isolated from Lactococcus lactis subsp. cremoris Wg2. It has a molecular weight of 70 kD ± 5 kD; an isoelectric point of 4.1 ± 0.4; a substrate range which includes peptides having five or more amino acids, metenkaphalin, bradykinin, glucagon, oxidized insulin $\beta$-chain and neurotensin, and excludes peptides having less than 5 amino acids, and $\alpha$-, $\beta$- and $\varkappa$-casein; a hydrolysis activity dependent of $Co^{2+}$; a temperature optimum at 30-38°C and a pH optimum at 6.0-6.5.

EP 0 522 203 A1

This invention relates to a protein originating from lactic acid bacteria, which protein is in isolated or purified form, i.e. in a substantially pure condition compared to the condition in which it is present in lactic acid bacteria in vivo. More in particular, the invention relates to a protein from lactic acid bacteria which is an enzyme having peptidase activity.

Furthermore, the invention relates to genetically transformed organisms, in particular microorganisms (bacteria, but also fungi and yeasts), which are capable of expression of the relevant protein (i.e. capable of forming the protein); to nucleotide sequences (in particular DNA) coding for the protein; to a recombinant polynucleotide, more in particular recombinant DNA comprising genetic information coding for the protein, as well as to the use of such a recombinant polynucleotide as a transformation marker; to a process for preparing the protein by isolation from an organism in which it is expressed, e.g., from lactic acid bacteria; to the use of the protein for preparing polyclonal and monoclonal antibodies against it (i.e. having affinity and/or specificity for the protein), as well as to such antibodies per se and to their use for detecting, removing and/or isolating the protein; to a process for preparing foods, such as cheese and other dairy products, meat products and protein hydrolysates, in which the protein having a peptidase enzyme activity is used, as well as to the foods thus obtained.

Lactic acid bacteria, more in particular lactic acid Lactococci such as Lactococcus lactis subsp. cremoris, lactis and diacetylactis, as well as other lactic acid bacteria such as Leuconostoc species, are largely used in the food industry, in particular in the dairy industry for preparing fermented milk products such as cheese. By producing lactic acid from lactose the food product is acidified, thus protecting the food product against decay. The bacteria are also involved in the development of flavour and taste of the food product. In this connection an important process is the breakdown of proteins.

For growth and for lactic acid production the bacteria require a nutrient medium providing essential growth factors such as vitamins and amino acids. Milk is a suitable nutrient medium and enables growth to high cell densities. For this purpose, however, the content of free amino acids in milk is insufficient, so that as far as their need for amino acids is concerned the bacteria depend on their proteolytic system for the breakdown of milk proteins, in particular casein. The proteolytic system of lactic acid bacteria also remains active after terminated growth, e.g., in the cheese ripening phase in which the breakdown of casein to peptides and then amino acids contributes to the flavour and taste development of the cheese.

The proteolytic system of lactococci comprises different proteinases and different peptidases. The proteinases of Lactococcus lactis hydrolyse proteins like $\beta$-casein into several oligopeptides of five or more amino acids. They are localized in the cell wall of the bacteria, whereas the peptidases which are responsible for further degradation of the formed oligopeptides to smaller peptides and amino acids may be present in the medium, in the cell wall, and within the cell.

Several peptidases of lactic acid bacteria have been isolated and characterized. T.-R. Yan et al., Eur. J. Biochem. 163, 1987, 259-265, and Appl. Environ. Microbiol. 53, 1987, 2296-2302, describe endopeptidases isolated from Streptococcus cremoris H61 which hydrolyse large casein fragments into smaller peptides which can then be degraded further by aminopeptidases. Specific X-prolyl-dipeptidylaminopeptidases degrade the proline-rich peptides while dipeptidases, tripeptidases, and prolidases release free amino acids and thereby finally complete the degradation of casein.

In the Dutch cheese industry Lactococcus lactis subsp. cremoris is a most important species of lactic acid bacteria. A good insight into the proteolytic system of lactococci such as L. lactis subsp. cremoris may contribute to an improvement of the starter cultures and to a better control of the ripening process, e.g., thus realizing accelerated cheese ripening or preventing development of a bitter taste (formation of bitter peptides). Much basic information is given in the thesis by R. Otto entitled "An ecophysiological study of starter streptococci", State University of Groningen (1981) and in the thesis by J. Hugenholtz entitled "Population dynamics of mixed starter cultures", State University of Groningen (1986). The latter thesis describes a study into the proteinases of L. lactis subsp. cremoris Wg2, and the thesis by A. van Boven entitled "The uptake and hydrolysis of peptides by S. cremoris" describes a further study into the proteolytic system of L. lactis subsp. cremoris Wg2.

Up till now there have been isolated and biochemically characterized three peptidases from L. lactis subsp. cremoris Wg2: a dipeptidase (A. van Boven et al., Appl. Environ. Microbiol. 54, 1988, 43-49), a tripeptidase (B.W. Bosman et al., Appl. Environ. Microbiol. 56, 1990, 1839-1843) and an aminopeptidase (P.S.T. Tan and W.N. Konings, Appl. Environ. Microbiol. 56, 1990, 526-532). These 3 enzymes (in addition to a X-prolyl-dipeptidyl aminopeptidase which is not completely characterized yet) are all exopeptidases and show no endopeptidase-like activity. An endopeptidase activity is essential, however, for an effective and complete degradation of casein.

The present invention is based on a further study into the peptidases of Lactococcus lactis subsp. cremoris Wg2, which resulted in the isolation and characterization of a new specific endopeptidase which is distinctly different from the known endopeptidases described by Yan et al. The endopeptidase was purified from a crude cell extract of L. lactis subsp. cremoris Wg2 by a procedure which included Diethylaminoethane (DEAE) Sephacel Column Chromatography, Phenyl Sepharose Chromatography, Hydroxylapatite Chromatography and Fast Performance Liquid Chromatography (FPLC) over an Anion Exchange Column and a Hydrophobic Interaction Column. Gel filtration and Sodium Dodecyl Sulfate Polyacrylamide Gel Electrophoresis indicated a molecular mass of the purified enzyme of 70 kD. The endopeptidase shows hydrolysis activity with respect to several oligopeptides which are degraded into various tetra-, tri- and di-peptides. The endopeptidase has no aminopeptidase, carboxypeptidase, dipeptidase or tripeptidase activities. It is optimally active at pH 6.0 to 6.5 and in a temperature range of 30 to 38°C. The enzyme is inactivated by the chemical agents 1,10-phenanthroline, ethylenedinitrilo-tetraacetate (EDTA), $\beta$-mercaptoethanol and phenylmethylsulfonyl fluoride, and is inhibited by $Cu^{2+}$ and $Zn^{2+}$. The ethylene-dinitrilotetraacetate or 1,10-phenanthroline treated enzyme can be reactivated by $Co^{2+}$. Immunoblotting with specific antibodies raised against the purified endopeptidase indicated that the enzyme is also present in other Lactococcus ssp., as well as in Lactobacillus ssp. and Streptococcus thermophilus ssp.

The novel endopeptidase according to the invention differs from the endopeptidases LEP I and LEP II reported by Yan et al., even though certain properties of the enzymes are similar such as inactivation by EDTA and the action towards certain peptides.

Whereas the present endopeptidase is a monomer with a molecular weight of 70 kD, LEP I derived from L. lactis subsp. cremoris H61 has a molecular weight of 98 kD and LEP II appears to be a dimer with a molecular weight of 80 kD. Differences are also observed with respect to the effects of various metal ions like $Zn^{2+}$ on the peptidase activity. $Zn^{2+}$ inhibits the activity of the present endopeptidase completely, while the LEP I activity is not affected. The metal-depleted LEP I activity can be restored by $Mn^{2+}$, while $Zn^{2+}$ can even reactivate the EDTA-treated LEP II activity. Also the pH-optima and the isoelectric points (pI) of the three enzymes differ: LEP I has its optimum at pH 7-7.5 and an isoelectric point of 5.1, whereas the present endopeptidase has a pH optimum at about 6.3 and a pI of 4.1. The enzymes also have different substrate specificities. LEP I cannot hydrolyse Glucagon and Oxidized insulin $\beta$-chain, in contrast to the present endopeptidase. Finally, the amino acid compositions and the N-terminal amino acid sequences of LEP II and the present endopeptidase differ significantly.

The novel endopeptidase may be used to prevent the development of an undesirable bitter taste during cheese ripening. Actually, it has been found that after casein breakdown some L. lactis subsp. cremoris strains produce different grades of bitterness, apparently as a result of the accumulation of peptides having a high content of hydrophobic amino acids which are only slowly broken down by the peptidases of the bacteria present in the starter. The use of the present endopeptidase could mitigate this problem.

Furthermore, the endopeptidase may be used for other purposes, such as clarification of beer and beverages, and it may of course also be used in DNA technology in a wide sense.

The invention is first embodied in the endopeptidase itself, i.e. a protein in isolated or purified form, which protein originates from lactic acid bacteria, characterized in that the protein has a $Co^{2+}$ dependent endopeptidase enzyme activity; a substrate range which includes peptides having five or more amino acids, metenkaphalin, bradykinin, glucagon, oxidized insulin $\beta$-chain and neurotensin but excludes peptides having less than five amino acids and $\alpha$-, $\beta$- and $x$-casein; a molecular weight of 70 kD ± 5 kD; an isoelectric point of 4.1 ± 0.4; a temperature optimum at 30-38°C and a pH optimum at 6.0-6.5.

The endopeptidase may be characterized further by the following amino acid sequence at the N-terminal end: Thr-Xaa-Ile-Gln-Asp-Asp-Leu-Phe-Ala-Thr-Val-Asn-Ala-Glu-Lys-Leu-, wherein Xaa stands for an unknown amino acid.

Although the endopeptidase which has actually been isolated and characterized according to the experimental part herein originates from one specific organism, namely the strain L. lactis subsp. cremoris Wg2, the invention also comprises the corresponding endopeptidases of other lactic acid bacteria, since these have corresponding properties and can be isolated and purified from the bacteria in a similar manner. By "corresponding endopeptidases" is not meant that the peptidases are exactly equal, i.e. have the same amino acid sequence, but is rather meant that they satisfy the above characterization of the endopeptidase.

A more specific embodiment of the invention is concerned with the endopeptidase originating from lactococci, in particular Lactococcus lactis subsp. cremoris, most preferably L. lactis subsp. cremoris Wg2.

The words "in isolated or purified form" and "originating from" do not mean to limit the invention to endopeptidases that have been isolated from their natural environment. In fact, the invention creates the possibility of tracing the endopeptidase gene and then realizing production of the endopeptidase in transformed cells or organisms by means of recombinant DNA technology. For this purpose, e.g., the amino acid sequence of the endopeptidase could be determined (a part of the amino acid sequence has been determined already). On the basis thereof, corresponding DNA sequences may be derived by means of the genetic code, after which suitable DNA probes may be synthesized with which endopeptidase messenger RNA (mRNA) can be detected and isolated. This mRNA may be used in standard methods for preparing copy DNA (cDNA) using enzymes such as reverse transcriptase and DNA polymerase. This cDNA may be cloned by means of suitable vectors (mostly plasmids or phages) and expressed in suitable host cells, from which the endopeptidase produced can be easily recovered. Similarly, the DNA probes can be used to locate the endopeptidase gene in the genome of the bacteria and the gene may then be used in standard methods for the transformation of suitable hosts and the expression therein of the gene.

On the basis of such genetic work, there can also be constructed a marker plasmid which comprises the endopeptidase gene fused with another desired gene. By a transformation of such a plasmid into an endopeptidase-less host an expression of the fusion product can be obtained, for which it can be easily screened by means of, e.g., a specific endopeptidase substrate such as metenkaphalin or by means of polyclonal or monoclonal antibodies against the endopeptidase. Such a marker plasmid may be of great significance to the food industry because the plasmid is derived from a bacterial species generally regarded as safe (i.e. a GRAS organism).

The endopeptidase according to the invention may be obtained by an isolation thereof from a cell mass, a culture medium or a secretion product of lactic acid bacteria, in which the endopeptidase is naturally expressed, or from a cell mass, a culture medium or a secretion product of an organism which, by means of genetic engineering, has acquired the ability of expressing the endopeptidase.

The invention is further also embodied in organisms, in particular microorganisms, which, by means of genetic engineering, have acquired either the ability foreign to them of expressing an endopeptidase according to the invention or the ability of a stronger expression of an endopeptidase according to the invention than they naturally possess.

The invention is also embodied in a recombinant polynucleotide, in particular recombinant DNA, comprising genetic information coding for an endopeptidase according to the invention. This recombinant polynucleotide may consist exclusively of the gene coding for the endopeptidase or may also comprise regulation sequences such as, e.g., a suitable transcription promoter, and/or a vector part.

The invention further comprises the use of such a recombinant polynucleotide as a transformation marker in which a successful transformation of an endopeptidase-negative organism is concluded from expression of the endopeptidase for which the polynucleotide comprises the genetic information, which expression is detected by means of a suitable substrate of the endopeptidase or by means of polyclonal or monoclonal antibodies having affinity and/or specificity for the endopeptidase. The invention also includes such a use as a transformation marker in the form of a plasmid containing the endopeptidase as a gene, which gene is inactivated by the introduction of another desired gene.

The invention further comprises a process for preparing an endopeptidase according to the invention, which process is characterized in that a crude cell extract of an organism, in particular a microorganism, in which the endopeptidase is expressed, such as L. lactis subsp. cremoris Wg2, is subjected to an isolation procedure which may include Diethylaminoethane Sephacel Column Chromatography, Phenyl Sepharose Chromatography, Hydroxylapatite Chromatography and Fast Performance Liquid Chromatography over an Anion Exchange Column and a Hydrophobic Interaction Column, in which in each chromatography step the eluate fractions having endopeptidaselike hydrolysis activity, in particular hydrolysis activity with respect to metenkaphalin, are selected.

Moreover, the invention creates the possibility to produce antibodies against the endopeptidase and then to use them to remove or isolate the endopeptidase from its natural environment or from the above transformed cells or organisms.

The invention is therefore also embodied in the use of the novel endopeptidase for preparing polyclonal or monoclonal antibodies having affinity and/or specificity for the endopeptidase, in these polyclonal and monoclonal antibodies themselves, and in their use for detecting the endopeptidase in, or removing or isolating it from a mixture containing the endopeptidase.

Of course, the invention is further embodied in a process for preparing foods such as cheese, whippable products and meat products, using an endopeptidase and/or an organism according to the invention. Herein specific hydrolysis properties of the endopeptidase can be used, e.g., to inhibit the development of a bitter flavour or to realize accelerated cheese ripening. The process may also have the advantage of lower production costs, e.g., because less starter is required. The foods obtained by this process are also comprised by the invention, in particular if they are distinguished from well-known products by a higher quality (better taste or, more in general, better organoleptic properties).

The invention will hereinafter be explained by means of the experimental part.

## Materials and methods

### Preparation of a cell extract

In the experiments the bacterial strain L. lactis subsp. cremoris Wg2 was used. The organism was routinely maintained in 10% (wt/vol) sterile reconstituted skim milk containing 0.1% (wt/vol) tryptone, and stored at a temperature of -20°C. L. lactis subsp. cremoris Wg2 was grown on MRS broth (J.C. de Man et al., J. Appl. Bacteriol. 23, 1960, 130-135) at a controlled pH of 6.3 in a 5 liter fermenter. Cells were harvested at an O.D. ($A_{660nm}$) of 1.9 by centrifugation at 7000 x g for 15 min at 4°C, washed in 400 ml 50 mM potassium phosphate (KPi; pH 6.0) and resuspended in 50 ml 20 mM KPi (pH 6.0). Cells were disrupted by French Press (Aminco, Silver Spring, Maryland, USA) at 4°C. Cell free extract was obtained by centrifugation of the disrupted cells for 20 min at 17,500 x g at 4°C.

### Enzyme Assays

Hydrolysis of peptides was detected by Thin Layer Chromatography (TLC). Endopeptidase activity was assayed as follows: reaction mixture containing 1.25 mM metenkaphalin (Tyrosyl-glycyl-glycyl-phenylalanyl-methionine) in 20 mM Tris/HCl pH 7.0 was incubated with an appropriate amount of enzyme for 15 min at 30°C. Samples (50 µl) were taken and the reaction was stopped by adding 10 µl 30% acetic acid and cooling the mixture to 4°C. The mixture (10 µl) was then spotted on a precoated silica gel 60 plate having a layer thickness of 0.25 cm (Merck, Darmstadt, Germany) and TLC was performed as described by P.S.T. Tan and W.N. Konings. In some cases 0.05% fluorescamine in 99% acetone was used to stain the gels. Peptides and amino acids became visible in UV-light.

One unit (1 U) of the endopeptidase activity was defined as 1 µmol metenkaphalin hydrolysed per min per mg protein at 30°C and pH 7. Metenkaphalin has proven to be a very good substrate for the screening of endopeptidase activity.

### DEAE-Sephacel Column Chromatography

A DEAE-Sephacel column (Pharmacia LKB Biotechnology, Uppsala, Sweden; 1.6 x 20 cm) was equilibrated with 20 mM KPi (pH 6.0) containing 0.1 M NaCl. The cell extract obtained from L. lactis subsp. cremoris Wg2 was diluted with 20 mM KPi to the same ionic strength as the buffer used for equilibrating the DEAE-Sephacel column and was then applied to the column. The ionic strength was measured with a Radiometer, Copenhagen, Denmark. After washing the column with two volumes of equilibration buffer, the enzyme was eluted (54 ml/h) with a linear gradient of 0.1 M to 0.4 M NaCl in 20 mM KPi. Fractions of 6 ml were collected and tested for endopeptidase activity. Fractions with the highest enzyme activities were combined.

### Phenyl Sepharose Column Chromatography

A Phenyl-Sepharose (CL 4B) column (Pharmacia LKB Biotechnology, Uppsala, Sweden; 1.8 x 8.5 cm) was equilibrated with 20 mM Tris/HCl pH 6.0 containing 4 M NaCl. NaCl was diluted with the combined enzyme fractions to the same osmolarity as the equilibration buffer. Subsequently, the enzyme solution was applied to the column. After washing of the column with two volumes of equilibration buffer, the enzyme was eluted (30 ml/h) with a linear gradient of 4 M NaCl to 0 M NaCl in 20 mM Tris/HCl pH 6.0. Fractions of 7.5 ml were collected and tested for endopeptidase activity. Fractions with the highest enzyme activities were combined.

### Chromatography on Hydroxylapatite

The enzyme fractions obtained by Phenyl-Sepharose chromatography were applied to a hydroxylapatite column (1.5 x 6.3 cm) that had been preequilibrated with 0.01 M Na-phosphate (NaPi) (pH 6.0). The column was washed with 2 volumes of the same buffer and the enzyme was eluted with a linear gradient of NaPi from 0.01 M to 0.4 M at pH 6.0. The flow rate was 40 ml/h. The fractions were tested for endopeptidase activity and the most active fractions were pooled.

### Second Anion Exchange Chromatography

For Fast Protein Liquid Chromatography a Mono Q HR 5/5 column was used (Pharmacia, Uppsala, Sweden) which was pre-equilibrated with 20 mM Tris/HCl (pH 6.0) containing 0.1 M NaCl. The combined fractions of the hydroxylapatite chromatography were then applied to the column. The enzyme was eluted with a linear gradient from 0.1 M to 0.3 M NaCl in 20 mM Tris/HCl (pH 6.0) at a flow rate of 1 ml/min. Fractions were tested for endopeptidase activity and the most active fractions were combined.

Second Hydrophobic Interaction Chromatography

Fast Protein Liquid Chromatography with a Phenyl-Superose HR 5/5 column (Pharmacia, Uppsala, Sweden) was done after preequilibration of the column with 20 mM Tris/HCl (pH 6.0) containing 4 M NaCl. The same molarity of NaCl was added to the active mono Q fractions before they were applied to the column. The enzyme was eluted with a linear gradient from 4 M to 0 M NaCl in 20 mM Tris/HCl pH 6.0 at a flow rate of 0.5 ml/min. The fractions were tested for endopeptidase activity and pooled.

High Performance Liquid Chromatography (HPLC)

HPLC was performed as described by B.W. Bosman et al. Purified endopeptidase (100 $\mu$l containing 25 $\mu$g protein) was injected onto an Ultrapac TSK G3000 SW gel filtration column (7.5 x 600 mm; Pharmacia LKB Biotechnology). The molecular weight of the protein was estimated by using the low-range protein standards (Pharmacia LKB Biotechnology). The single peak was tested for metenkaphalin hydrolysis.

SDS-PAGE

Sodium dodecyl sulfate (SDS) polyacrylamide gel electrophoresis (PAGE) was carried out as described by U.K. Laemmli and K. Faure, J. Mol. Biol. 80, 1973, 575-599. The protein samples were mixed 4 : 1 with sample buffer (0.05 M Tris/HCl pH 6.8, 10% SDS, 22% glycerol, 10% $\beta$-mercaptoethanol, 0.18% bromphenol blue) and applied to the gels. The molecular weights of the protein bands were estimated by using the low-range SDS-PAGE standards (Bio-Rad Laboratories, Richmond, California). The gels were stained either with Coomassie Brilliant Blue or with silver according to the method of W. Wray et al., Anal. Biochem. 118, 1981, 197-203.

Isoelectric Focussing

For isoelectric focussing (IEF) on slab gels a Pharmacia Phast System was used with a ready-to-use 5% polyacrylamide gel containing Pharmalyte 3-9 (Phastgel, Pharmacia). The isoelectric point was determined using the reference proteins from the broad pI-range calibration kit (Pharmacia, Uppsala, Sweden). The gels were automatically stained according to the Phast System with Coomassie brilliant blue.

Effect of divalent cations and chemical agents on the enzyme activity

Purified enzyme (99 $\mu$g) was preincubated for 30 min at 4°C with EDTA (1 mM) in Tris/HCl pH 6.0. After dialyzing for 24 h against the same buffer, the enzyme solution was incubated with several concentrations of divalent cations for 30 min at 4°C before adding metenkaphalin to start the reaction. The reaction was stopped and the reaction products analysed as described above. An appropriate amount of enzyme was incubated with chemical agents (1 mM) for 30 min at 4°C and the effect on the enzyme activity was studied.

Anti-endopeptidase serum

The antiserum to the enzyme was raised in rabbits after immunization by the following protocol. A sample (100 $\mu$l; 0.5 mg protein/ml) of purified enzyme was injected with complete Freund adjuvant. After one month a booster injection of 50 $\mu$g purified enzyme was given. The rabbits were bled 7 and 14 days after each booster injection. Serum was prepared from blood by centrifugation at 10,000 x g for 10 min.

Immunoblotting

After PAGE of samples containing the crude extracts of different Lactococcus lactis ssp., Lactobacillus subsp. bulgaricus var. diacetylactis and Streptococcus thermophilus strains or purified endopeptidase, the proteins separated on the gel were transferred to polyvinylidene difluoride (PVDF) sheets with a semi-dry electroblotter (Ancos, Denmark) by the technique of J. Kyhse-Andersen, J. Biochem. Biophys. Meth. 10, 1984, 203-209. The PVDF-membrane was saturated with 1% skimmed milk and was then incubated with 1/7000 diluted rabbit anti-endopeptidase serum. Subsequently, the membrane was incubated with 1/1000 diluted alkaline phosphatase-conjugated goat-rabbit immunoglobulin serum (Sigma, St. Louis, MO, USA).

After washing with 10 mM Tris/HCl pH 8.0 containing 150 mM NaCl and 0.05% Tween-20 (TBST), the enzyme antibody complex was visualized by incubating the membrane in an alkaline carbonate buffer pH 9.8 containing 1% (v/v) nitro blue tetrazolium (NBT, 30 mg/ml) in 70% dimethylformamide and 1% (v/v) 5-bromo-4-chloro-3-indolyl phosphate (BCIP, 15 mg/ml) in 100% dimethylformamide.

Crossed Immuno Electrophoresis (CIE)

CIE was carried out as described by J. van der Plas et al., J. Bacteriol. 153, 1983, 1027-1037. The gels were run at 30 V/cm for 90 min in the first dimension and at 40 V/cm for 18-24 h in the second dimension. CIE was performed of the purified enzyme (80 $\mu$g protein/ml) against antibodies (0.5 mg protein/ml) raised against the cell extract of L. lactis subsp. cremoris Wg2 described by J. Hugenholtz et al., Appl. Env. Microbiol. 48, 1984, 1105-1110, and of (0.32 mg protein/ml) cell free extract from L. lactis subsp. cremoris Wg2 against antibodies (17.8 mg protein/ml) raised against the purified endopeptidase. The gels were stained with Coomassie Brilliant Blue to show the precipitate.

Casein hydrolysis

An appropriate amount of purified enzyme was added to a mixture of $\alpha$, $\beta$ and $x$ casein (1.5 mg/ml) in a Tris/HCl pH 7 buffer. After overnight incubation at 30°C, the samples were boiled for 5 min in sample buffer as described above. Proteins were separated by electrophoresis on a 12.5% SDS-PAA gel.

Protein determination

Protein concentrations were determined by the method of O.H. Lowry et al., J. Biol. Chem. 193, 1951, 265-275, with bovine serum albumin as standard.

Amino acid and N-terminal Sequence Analysis

The amino acid analysis and N-terminal amino acid sequence analysis were carried out as described by P.S.T. Tan and W.N. Konings.

Chemicals

All chemicals used were commercially available reagent grade substances.

RESULTS

Enzyme purification

Step a. After DEAE Sephacel column chromatography of the crude cell extract (400 ml) from L. lactis subsp. cremoris Wg2 the highest metenkaphalin-hydrolyzing activities were found in the NaCl gradient elution fractions 90 to 120 between 0.18 and 0.24 M NaCl. These fractions were combined and NaCl was added to a final concentration of 4 M NaCl before these fractions were applied onto a Phenyl Sepharose column.

Step b. The combined fractions (186 ml) were applied onto a Phenyl-Sepharose column. After elution from 4 M to 0 M NaCl metenkaphalin hydrolyzing fractions were found at 0.9 to 0.6 M NaCl.

Step c. The pooled fractions (90 ml) were applied onto a hydroxylapatite column. After elution with a 0.1 M to 0.4 M NaPi gradient, metenkaphalin hydrolyzing activities were obtained in fractions at 0.21 to 0.25 M NaPi.

Step d. The combined endopeptidase fractions (3x 25 ml) were applied onto a Mono Q column. Elution of the column with a 0.1 M to 0.3 M NaCl gradient resulted in metenkaphalin hydrolyzing activities in the fractions at 0.23 M to 0.27 M NaCl. Fractions were pooled and NaCl was added to a final concentration of 4 M NaCl before these fractions were applied onto a Phenyl Superose column.

Step e. For the final step 2 ml of the combined endopeptidase fractions were applied onto a Phenyl Superose column. The pure enzyme was eluted at 1.6 M at a retention time of 16 to 24 min. The enzyme purification is summarized in Table 1. A total yield of the purification procedure of 17.4% and a purification factor of 678 were estimated. After the last purification step a single peak was eluted.

Several peptidase activities are involved in the complete hydrolysis of metenkaphalin into amino acids. After the third purification step the tripeptide Tyrosyl-glycyl-glycine was not further hydrolyzed indicating that tripeptidase activity and aminopeptidase activity had been removed. However, dipeptidase activity was still present as indicated by the hydrolysis of Phe-Met. The fractions obtained from the Mono Q column hydrolyzed metenkaphalin only into Tyr-Gly-Gly and Phe-Met indicating that only the endopeptidase activity is retained. However, SDS-PAGE showed that the fractions were still contaminated with other proteins. These contaminations were removed in the last purification step. After every purification step the specific activity increased (Table 1).

### Molecular weight and isoelectric point

The molecular mass of the endopeptidase was estimated to be 70 kD by HPLC with a TSK G3000 SW column and by 10% SDS-PAGE. On SDS-PAGE the purified enzyme gave one band with silver as well as with Coomassie Brilliant Blue staining. A treatment of the purified enzyme with $\beta$-mercaptoethanol after boiling for 5 min or under native conditions yielded the same results indicating that the enzyme consists of a single polypeptide.

The pI of the enzyme was estimated to be 4.3 by isoelectric focussing.

### Immunoblotting

Polyclonal antibodies against the purified protein were obtained. The results of immunoblottings with the purified enzyme and cell free extract from strain Wg2 confirmed the purity of the enzyme. Also no cross-reaction between the antiserum and other proteins in the crude extract could be observed. Immunoblotting with cell extracts of L. lactis subsp. cremoris H61, p8-2-47, E8, AM2, SK11 and HP and L. lactis subsp. lactis ML1 and ML3, as well as Streptococcus thermophilus A147 and Lactobacillus bulgaricus B131 also revealed a 70 kD band when incubated with the antiserum raised against the endopeptidase.

### Crossed Immuno Electrophoresis (CIE)

CIE with the protein obtained after the last purification step (10 $\mu$l of the most purified protein was applied) was performed in 1% agarose gel, which contained in the second dimension 0.5 mg protein/ml antibodies raised against whole cells of L. lactis subsp. cremoris Wg2. Also CIE was performed with 4 $\mu$l cell free extract of L. lactis subsp. cremoris Wg2 (0.32 mg protein/ml), which contained in the second dimension 17.8 mg/ml antibodies raised against the purified endopeptidase. After staining the gel with Coomassie Brilliant Blue, the purified sample as well as the cell free extract showed only one precipitation line indicating that the purified fraction contained only one protein and that the antibodies directed against the endopeptidase were specific.

These results clearly indicate that the endopeptidase is purified to homogeneity. Further characterization was done with the pure endopeptidase.

### Temperature and pH dependence of the endopeptidase activity

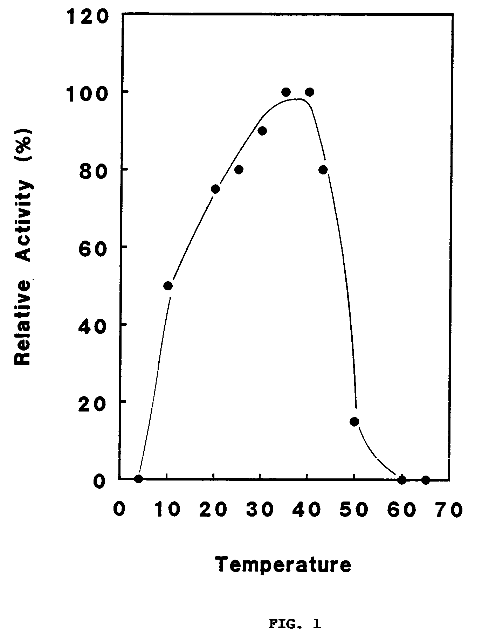

The effect of the temperature on the endopeptidase activity was measured in a range of 4 to 60°C. The enzyme mixture was equilibrated for 5 min at the temperatures tested before the addition of metenkaphalin. The temperature optimum of the endopeptidase for metenkaphalin hydrolyzing activity was found to be between 30 and 38°C (Fig. 1).

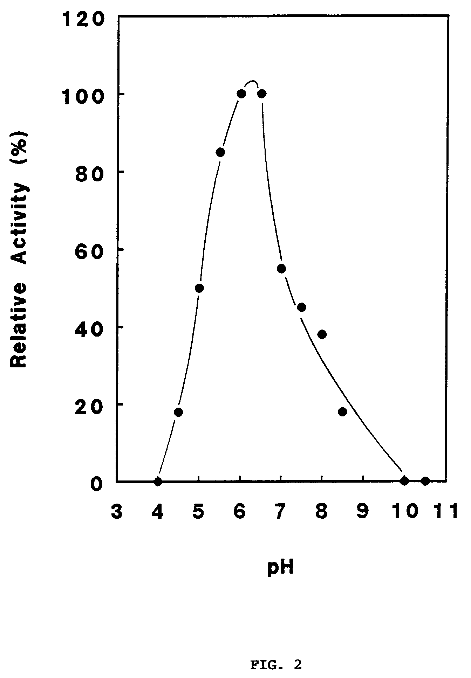

The effect of the pH on the enzyme activity was examined in the range from pH 4 to 10 by using a buffer consisting of 20 mM each of malic acid, 2-(N-morpholino)ethanesulphonic acid (MES), 4-(2-hydroxyethyl)-1-piperazine-ethanesulfonic acid (HEPES), and boric acid adjusted to the appropriate pH values. The optimum pH for hydrolyzing metenkaphalin was found to be between pH 6.0 and 6.5. At pH values below 4.5 and above 10.0 no hydrolysis of metenkaphalin was detected (Fig. 2).

### Substrate specificity

The hydrolytic effect of the enzyme on several peptides is summarised in Table 2. The products of the hydrolysis were analysed by thin layer chromatography (TLC). The purified endopeptidase showed a broad substrate specificity. Peptides smaller than 5 amino acid residues were not hydrolyzed. The largest peptide which was hydrolyzed by the endopeptidase was the oxidized $\beta$-insulin chain. No hydrolysis of $\alpha$, $\beta$ or $\varkappa$-casein was detected even after overnight incubation with the purified enzyme.

Effect of various chemical agents

Table 3 shows that the endopeptidase activity was inhibited by the chelating agents EDTA and 1,10-phenanthroline (1.0 mM). The enzyme was also inhibited by the serine proteinase inhibitor phenylmethylsulfonylfluoride (PMSF) and by the disulfide reducing agent $\beta$-mercaptoethanol (1%), but not by dithiothreitol (DTT). The sulfhydryl reagents p-chloromercuribenzoate (pCMB) and p-chloromercuribenzenesulfonate (pCMBS) and O-(3-hydroxymercuri-2-methoxypropyl)-carbamylphenoxyacetate (mersalyl) did not have an effect on the activity. The inhibition of the enzyme by $\beta$-mercaptoethanol appeared to be irreversible; oxidizing reagents like ferricyanide, plumbagine and oxidized gluta thione (up to 5 mM) could not restore the enzyme activity.

Effect of metal ions on enzyme activity

Treatment of the enzyme (4 $\mu$g protein) with 1 mM of $Cu^{2+}$ or $Zn^{2+}$ inhibited the enzyme activity completely. Other cations like $Co^{2+}$ and $Mn^{2+}$ (1 mM) had no effect on the enzyme activity (Table 4). Enzyme activity which was inhibited by EDTA or 1,10-phenanthroline could be restored by 50 to 300 $\mu$M $Co^{2+}$ and not by other divalent cations (Table 5). This indicates that the enzyme is a $Co^{2+}$ dependent metallo-peptidase.

Amino acid composition and N-terminal sequence

The amino acid composition of the purified endopeptidase (Table 6) shows a high content of serine and glycine residues. Sulfur-containing amino acids like cystein and methionine are present at low concentrations. The molecular mass as calculated from the nearest integers of the amino acids was found to be 66 kD. The partial amino-terminal sequence of the endopeptidase was determined up to the 16th residue as Thr-Xaa-Ile-Gln-Asp-Asp-Leu-Phe-Ala-Thr-Val-Asn-Ala-Glu-Lys-Leu-.

Discussion of the Figures

Fig. 1. Effect of the temperature on the activity of the purified peptidase in 20 mM Tris/HCl (pH 7). The relative endopeptidase activity was measured as described above.

Fig. 2. Effect of the pH on the endopeptidase activity measured in 20 mM malic acid - 20 mM MES - 20 mM HEPES - 20 mM boric acid and adjusted to different pH values with KOH. The relative endopeptidase activity was measured as described above.

Table 1

| Purification of an endopeptidase from L. lactis subsp. cremoris Wg2. | | | | | |
|---|---|---|---|---|---|
| Purification Step | Total Protein (mg) | Total[a] Activity | Yield (%) | Specific[b] Activity | Purification (fold) |
| Cell Extract | 6,640 | 278.9 | 100 | 0.042 | 1 |
| DEAE-Sephacel | 902.1 | 387.9 | 139 | 0.430 | 10 |
| Phenyl-Sepharose | 102.6 | 112.4 | 40.3 | 1.096 | 26 |
| Hydroxyl Apatite | 38.5 | 96.3 | 34.5 | 2.500 | 60 |
| Mono Q | 2.4 | 48.5 | 17.4 | 20.208 | 481 |
| Phenyl Superose | 1.7 | 48.4 | 17.4 | 28.470 | 678 |

[a] Total Activity is expressed as micromoles of metenkaphalin hydrolysed per min.
[b] Specific Activity is expressed as micromoles of metenkaphalin hydrolysed per mg of protein per min.

9

Table 2. Substrate specificity of an endopeptidase from L. lactis subsp. cremoris Wg2. Hydrolysis of the peptides was analysed by Thin-Layer-Chromatography (TLC.) Hydrolysis of the chromogenic substrates[a] was also measured at $A_{410}$ and the hydrolysis of casein[b] was detected by SDS-PAGE.

| Substrate | Activity |
|---|---|
| Lys-pNA[a] | - |
| Gly-Pro-pNA[a] | - |
| Ala-Pro-pNA[a] | - |
| Bz-Gly-Pro | - |
| Bz-Gly-Lys | - |
| Z-Pro-Ala | - |
| Z-Phe-Ala | - |
| Gly-Gly | - |
| Leu-Leu | - |
| Phe-Met | - |
| Phe-Val | - |
| Tyr-Gly-Gly | - |
| Leu-Leu-Leu | - |
| Met-Gly-Gly | - |
| Leu-Gly-Gly | - |
| Gly-Val-Phe | - |
| Gly-Pro-Ala-Pro | - |
| Gly-Pro-Gly-Gly | - |
| $N_{succ}$-Ala-Ala-Ala-pNA[a] | - |
| $N_{succ}$-Phe-pNA[a] | - |
| $N_{succ}$-Ala-Ala-Pro-Leu-pNA[a] | - |
| Z-Leu-Gly-Gly-pNA | - |
| $Ala_5$ | - |
| $Ala_6$ | - |
| Metenkaphalin | + |
| β-Casomorphin | - |
| Bradykinin | + |
| Substance P | + |
| Glucagon | + |
| oxydized insulin β-chain | + |
| Neurotensin | + |
| α-Casein[b] | - |
| β-Casein[b] | - |
| κ-Casein[b] | - |
| β-Casein( 33- 48) | - |
| β-Casein(184-202) | + |
| β-Casein(203-209) | + |

+ : hydrolysis
- : no hydrolysis detectable

Table 3. Effect of potential inhibitors on the endopeptidase
activity. Purified enzyme (4 µg protein) was
preincubated for 10 min at 20°C with 1 mM chemical
agent. After addition of metenkaphalin the fractions
were incubated for 15 min at 30°C. The enzyme
activity was tested by Thin Layer Chromatography.

----------------------------------------------------------------

| Inhibitor | Inhibition |
|-----------|------------|
| None | − |
| pCMB | − |
| 1,10-phenanthroline | + |
| Mersalyl | − |
| DEPC | − |
| Ferricyanide | − |
| Phenylarsenoxide | − |
| N-Ethylmaleimide | − |
| EDTA | + |
| Cystine | − |
| pCMBS | − |
| Thorin | − |
| Plumbagin | − |
| Hydroxylamine | − |
| β-Mercaptoethanol | + |
| DTT | − |
| PMSF | + |

----------------------------------------------------------------

+ : Inhibition

− : No inhibition detectable.

Table 4. Effect of metal ions on the activity of endopeptidase.
Activity measurements were done by Thin Layer
Chromatrography.

| Metal Ion (1 mM) | Inhibition |
|---|---|
| None | − |
| $Ca^{++}$ | − |
| $Co^{++}$ | − |
| $Cu^{++}$ | + |
| $Mg^{++}$ | − |
| $Mn^{++}$ | − |
| $Zn^{++}$ | + |
| $Fe^{++}$ | − |

+ : no hydrolysis detected

− : hydrolysis.

Table 5. Reactivation experiments with endopeptidase treated
with EDTA and 1,10-Phenanthroline. Hydrolysis of the
substrate was detected by Thin Layer Chromatography.

| Concentration: | Metal Ion: | | | | | | |
|---|---|---|---|---|---|---|---|
| ($\mu$M) | $Mn^{++}$ | $Co^{++}$ | $Cu^{++}$ | $Ca^{++}$ | $Fe^{++}$ | $Mg^{++}$ | $Zn^{++}$ |
| 0 | − | − | − | − | − | − | − |
| 50 | − | + | − | − | − | − | − |
| 100 | − | + | − | − | − | − | − |
| 150 | − | + | − | − | − | − | − |
| 200 | − | + | − | − | − | − | − |
| 300 | − | + | n.d. | n.d. | n.d. | n.d. | − |
| 1000 | − | − | − | − | − | − | − |

+ : hydrolysis
− : no hydrolysis detected
n.d. : Not Determined.

Table 6.  The amino acid composition of endopeptidase. Arg and
          Trp residues of endopeptidase were not determined.
          The nearest integer is based on a molecular mass of
          66 kDa.

| Amino acid : | % of amino acid residues in enzyme : | nearest integer : |
|---|---|---|
| Asx | 8.78 | 58 |
| Glx | 7.97 | 52 |
| Ser | 10.25 | 68 |
| His | 2.00 | 13 |
| Gly | 13.46 | 89 |
| Thr | 5.54 | 37 |
| Ala | 9.36 | 62 |
| Arg | n.d. | n.d. |
| Tyr | 2.44 | 16 |
| Cys | 2.94 | 19 |
| Val | 4.54 | 30 |
| Met | 1.77 | 12 |
| Phe | 5.54 | 37 |
| Ile | 5.24 | 35 |
| Leu | 9.12 | 60 |
| Lys | 5.98 | 40 |
| Pro | 2.30 | 15 |
| Trp | n.d. | n.d. |

n.d. : not determined

**Claims**

1.  Protein in isolated or purified form, which protein originates from lactic acid bacteria, characterized in that the protein has a $Co^{2+}$ dependent endopeptidase enzyme activity; a substrate range which includes peptides having five or more amino acids, metenkaphalin, bradykinin, glucagon, oxidized insulin $\beta$-chain and neurotensin but excludes peptides having less than five amino acids and $\alpha$-, $\beta$- and $x$-casein; a molecular weight of 70 kD ± 5 kD; an isoelectric point of 4.1 ± 0.4; a temperature optimum at 30-38°C and a pH optimum at 6.0-6.5.

2.  Protein according to claim 1, originating from lactic acid lactococci.

3.  Protein according to claim 2, originating from the lactic acid lactococcus Lactococcus lactis.

4.  Protein according to claim 2, originating from the lactic acid lactococcus Lactococcus lactis subsp. cremoris.

5.  Protein according to claim 2, originating from the lactic acid lactococcus Lactococcus lactis subsp.

cremoris Wg2.

6. Protein according to any of claims 1-5, further characterized by the following amino acid sequence at the N-terminal end: Thr-Xaa-Ile-Gln-Asp-Asp-Leu-Phe-Ala-Thr-Val-Asn-Ala-Glu-Lys-Leu-, wherein Xaa stands for an unknown amino acid.

7. Protein according to any of claims 1-5, obtained by an isolation thereof from a cell mass, a culture medium or a secretion product of lactic acid bacteria in which the protein is naturally expressed, or from a cell mass, a culture medium or a secretion product of an organism which, by genetic engineering, has acquired the ability to express the protein.

8. Organisms, in particular microorganisms, which, by genetic engineering, have acquired either the ability foreign to them to express a protein according to any of claims 1-7 or the ability of a stronger expression of a protein according to any of claims 1-7 than they naturally possess.

9. Recombinant polynucleotide, in particular recombinant DNA, which comprises genetic information coding for a protein according to any of claims 1-7.

10. A process for preparing a protein according to any of claims 1-7, characterized in that a crude cell extract of an organism, in particular a microorganism, in which the protein is expressed, such as L. lactis subsp. cremoris Wg2, is subjected to an isolation procedure which may include Diethylaminoethane Sephacel Column Chromatography, Phenyl Sepharose Chromatography, Hydroxylapatite Chromatography and Fast Performance Liquid Chromatography over an Anion Exchange Column and a Hydrophobic Interaction Column, in which in each chromatography step eluate fractions having endopeptidase-like hydrolysis activity, in particular hydrolysis activity with respect to metenkaphalin, are selected.

11. The use of a protein according to any of claims 1-7 for preparing polyclonal or monoclonal antibodies which have affinity and/or specificity for the protein.

12. Polyclonal and monoclonal antibodies having affinity and/or specificity for a protein according to any of claims 1-7.

13. The use of polyclonal or monoclonal antibodies according to claim 12 for detecting, removing and/or isolating the protein.

14. The use of a recombinant polynucleotide according to claim 9 as a transformation marker, in which the expression of the endopeptidase for which the polynucleotide contains the genetic information is detected by means of an appropriate substrate of the endopeptidase or by means of polyclonal or monoclonal antibodies having affinity and/or specificity for the endopeptidase.

15. A process for preparing foods such as cheese, whippable products and meat products, characterized by using a protein according to any of claims 1-7 and/or an organism according to claim 8.

16. Foods such as cheese, whippable products and meat products, obtained using a protein according to any of claims 1-7 and/or an organism according to claim 8.

FIG. 1

FIG. 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,X | EUROPEAN JOURNAL OF BIOCHEMISTRY vol. 163, 1987, pages 259 - 265; TSONG-RONG YAN ET AL.: 'purification and characterization of a novel metalloendopeptidase from Streptococcus cremoris H61' * the whole document * * page 264, left column, paragraph 2 - right column, paragraph 3; table 5 * | 1,7 | C12N9/52 C12N15/57 C12N1/21 C12P21/08 A61K39/395 C12Q1/37 C12N15/65 A23C19/032 A23C19/06 |
| D,A | APPLIED AND ENVIRONMENTAL MICROBIOLOGY vol. 53, no. 10, October 1987, pages 2296 - 2302; TSONG-RONG YAN ET AL.: 'Purification and characterization of a substrate-size-recognizing metalloendopeptidase from Streptpcoccus cremoris H61' * the whole document * | 1,7,10, 15,16 | |
| A | NETHERLANDS MILK AND DAIRY JOURNAL. vol. 43, 1989, WAGENINGEN NL pages 327 - 345; H. LAAN ET AL.: 'Enzymes involved in the degradation and utilization of casein in Lactococcus lactis' * page 327, paragraph 2 - page 328, paragraph 2 * * page 341, paragraph 2 - page 342, paragraph 2 * | 1-4,7, 11-13 | TECHNICAL FIELDS SEARCHED (Int. Cl.5 ) C12N A23C |

The present search report has been drawn up for all claims

| Place of search | Date of completion of the search | Examiner |
|---|---|---|
| THE HAGUE | 17 JANUARY 1992 | MONTERO LOPEZ B. |

EPO FORM 1503 03.82 (P0401)